# EUROPEAN PATENT SPECIFICATION

(11) **EP 0 524 972 B1**
(45) Date of publication and mention of the grant of the patent: **21.06.1995**
(21) Application number: 91906945.0
(22) Date of filing: 19.03.1991
(51) Int. Cl.: A01N 31/02, A01N 31/04

(54) **INSECT REPELLENT LOTIONS AND SPRAYS**
INSEKTENVERTREIBENDE LOTIONEN UND SPRAYS
LOTIONS ET SPRAYS ANTI-INSECTES

(30) Priority: 09.04.1990 US 506471
(43) Date of publication of application: 03.02.1993
(73) Proprietor: PRIMAVERA LABORATORIES, Inc., New York, New York 10022 (US)
(72) Inventor: BELDOCK, Donald, T., New York, NY 10021 (US); BELDOCK, John, A., Washington, D.C. 20007 (US)
(74) Representative: Austin, Hedley William (GB)
(86) International application number: US9101857
(87) International publication number: WO9115118

(56) References cited:
- EP-B- 0 275 085
- CA-A- 1 230 826
- FR-A- 855 176
- FR-B- 2 622 103
- JP-A- 2 O67 202
- US-A- 4 193 986
- US-A- 4 478 853
- US-A- 4 774 081
- US-A- 4 829 092
- W.PERKOW 'DIE INSEKTIZIDE' 1968 , A.Hüthig, HEIDELBERG, DE
- DATABASE WPI Week 7611, Derwent Publications Ltd., London, GB; AN 76-19329X
- CHEMICAL ABSTRACTS, vol. 105, no. 4 Columbus, Ohio, US; abstract no. 29193n, L.BEKESI ET AL 'COMPOSITIONS FOR MODIFYING THE ORGANOLEPTIC THRESHOLD VALUE OF ODORANTS, AS WELL AS FOR REDUCING THE SENSITIVITY OF THE ODOR SENSING HUMAN/ANIMAL NERVES'
- CHEMICAL ABSTRACTS, Volume 104, No. 5, issued 3 February 1986 (03.02.86)(Columbus, Ohio, US), C. BARTLETT, "An olfactometer for measuring the repellent effect on chemicals on the stable fly stomoxys calcitrans (L.)33", Pestic. Sci. Volume 16, No. 5, pages 479-487, 1985, the Abstract No. 30390K.
- CHEMICAL ABSTRACTS, Volume 105, No. 23, issued 8 December 1986 (08.12.86)(Columbus, Ohio, US), N. SAIM et al., "Compounds from leaves of Bay (LAURUS nobilis L.) as repellents for Tribolium casteneum (HERBST) when added to Wheat flour", J. Stored Prod. Res. Volume 22, No. 3, pages 141-144, 1986, Abstract No. 204742Q.

## Description

### BACKGROUND OF THE INVENTION

The present invention relates generally to insect repellents in lotion and spray form. More particularly, the present invention relates to insect repellents for humans and animals which are particularly efficacious in repelling ticks carrying Lyme disease, as well as biting flies and triatomes (Chagas bugs). For purposes herein, the term "insect" is to be understood in its broadest sense to include ticks, Chages bugs, biting flies, etc.

Insects have long been carriers and spreaders of diseases as they not only feed on animals, but on humans as well. In North America, mosquitoes, ticks, and black flies are the three major groups of arthropods pestiforous to humans. While black flies and mosquitoes in North America are primarily a nuisance, a tick bite can be more serious. In particular, significant attention in the northeast United States recently has been focused upon the Lyme disease ticks (ixodes damini) which have spread in geographical area as well as in number, and which carry the potentially debilitating Lyme disease. Similarly, in warmer climates such as Latin America, a serious and potentially fatal malady known as Chagas' disease are carried by triatomes (Chagas bugs) which are active at night and feed on people as they sleep. In Africa, mosquitoes carry malaria.

In an attempt to repel insects, people have turned to widely marketed lotions and sprays (e.g. Cutters, DeepWoods Off, and Tick Garde) which contain N,N-diethyl-m-toluamide (DEET) as their active ingredient. While DEET is an effective repellent, it is not particularly pleasing in smell, it stings when applied, and its use has a number of harmful side-effects to humans. DEET is injurious to eyes, mucous membranes, and sensitive skin. In addition, because DEET is absorbed through the skin, toxic systemic reactions may result as well. For example, in August 1989, the New York State Department of Health investigated five reports of generalized seizures which were believed to be associated with the topical application of DEET. Other symptoms and maladies associated with repeated exposure to DEET have included irritability, confusion, insomnia, encephalopathy, and coma. As a result, cautionary statements regarding use of DEET have been issued by the Centers for Disease Control and the states of New York, Connecticut, New Jersey, and Utah.

The potential hazards of using a product with DEET as an active ingredient suggests that there exists a great need for a comparably repellent product that is not dangerous to its users. This is particularly true in light of the dramatic increase in the incidences of Lyme tick bites.

We have now developed an insect repellent formulation suitable for human use which alleviates the above problems, which comprises:
(a) a vehicle constituting up to 99.97% of the insect repellent, said vehicle being suitable for application to and use on a human body;
(b) 0.01% to 1% terpineol; and
(c) 0.01% to 1% citronella oil;
   together with one or more of rhodinol and geraniol, each of which, when present, is in an amount of 0.01% to 1%;
wherein all listed percentages (%) are by weight.

In a preferred embodiment, both the rhodinol and geraniol are present in the formulation according to the invention.

Essential oils and their constituents are used in the unrelated field of deodorants. JP51009729 describes a deodorant for animal husbandry comprising a mixture of herbal and arboreal essential oils (cypress oil, white-cedar oil, cedar oil, citronella oil and their constituents such as pinene, geraniol, citronellol, terpineol and a mixture thereof) together with wood vinegar and wood tar extracts.

HU36383 describes ethanol based deodorant compositions for deodorising air spaces, such as pet breeding areas or workplaces the compositions containing the multiple active ingredients rosephenone, phenylacetic acid, clove oil, vanillin, heliotropin, coumarin, citronella oil, diphenyl oxide, phenylethyl alcohol, eucalyptus oil, Parahex, lowender oil, mentharnyl acetate, sandol, terpinolene, isoraldeine, terpineol and dimethyl phthalate.

Preferred weight percentages of the active ingredients present in an insect repellent formulation according to the invention are substantially 0.06% terpineol, substantially 0.05% citronella oil, substantially 0.08% rhodinol and substantially 0.06% geraniol.

Preferably the terpineol, citronella oil, rhodinol and geraniol are actives present in the vehicle in relative weights of substantially 6:5:8:6.

In a preferred embodiment, the vehicle comprises any of the following: a cosmetic moisturiser lotion optionally containing a sun screen, a water based vehicle, or an alcohol based vehicle.

It may be preferred that the insect repellent formulation further comprises a fragrance which typically constitutes substantially 0.4% of the repellent formulation.

A preferred insect repellent formulation comprises a cosmetic moisturiser lotion base to which 0.06% terpineol, 0.05% citronella oil, 0.08% rhodinol and 0.06% geraniol are added as well as 0.4% fragrance formula HB-728, commercially available from Shaw Mudge & Company (all percentages being weight percentages). The preferred insect repellent (hereinafter referred to as TREO) is safe to use, has excellent repellency characteristics, and has a pleasant smell.

TREO was tested for efficacy against DEET products in several areas of North America and Central America in different habitats, at different times of year, and against a wide variety of biting arthropods, including disease vectors. Several different human subjects were used to reduce any effects of inter-individual differences in attractiveness to biting arthropods. In each trial with mosquitoes or other biting flies, each human subject had one arm treated with a repellent (DEET or TREO) and the other untreated, permitting simultaneous testing. Bites were counted on each arm below the shoulder. In trials with ticks on Nantucket, conventional 2' by 2' flannel flags were treated with TREO, a DEET product, or were left untreated. These flags were passed through vegetation harboring ticks.

Table 1 summarizes the results of the five main investigations:

**Table 1**

| Repellency of TREO compared to products containing 35-38& DEET and to untreated controls | | | |
|---|---|---|---|
| Arthropod/Sites | Treatment | | |
| | TREO | DEET | Untreated |
| Mosquito (FL & TX)¹ | 5 | 3 | 170 |
| Mosquito (Utah)¹ | 16 | 3 | 451 |
| Ticks (Mass)² | 0 | 0 | 20 |
| Ticks (laboratory)³ | 0.3 | 1.25 | 4.0 |
| Black Flies (Belize)¹ | 0 | 0 | 55 |

| | | | |
|---|---|---|---|
| ¹ Numbers of bites per treatment | | | |
| ² Numbers of ticks clinging to cloth | | | |
| ³ Numbers of ticks crossing a repellent barrier per minute | | | |

From Table 1 it is seen that TREO proved to be an excellent repellent against all arthropods tested. In tests with mosquitoes in winter and spring in Florida and Texas, both TREO and a DEET product reduced biting rates by approximately 97-98%. In summer tests in Utah, the DEET product was marginally more effective against the marshland mosquito Aedes dorsalis, but the products were equally effective against the common urban and suburban pest Cluex tarsalis, the vector of Western Equine Encephalitis. In Belize, TREO was an absolute repellent against simuliid black files, which are vectors of river blindness, one of the ten most important arthropod borne diseases in the world. Also, in unquantified trials not shown in Table 1, TREO provided extreme protection against mosquitoes and ceraptopogonid black flies (No-see-ums) in Belize.

The Massachusetts tick test of Table 1 was conducted on Nantucket Island, in the month of October when approximately sixty percent of the ticks on Nantucket carry Lyme disease. Ticks were collected using the conventional technique of "flagging": 2' x 2' sheets of white flannel cloth were tacked to wooden dowels, and then swept across the tips of low vegetation harboring active ticks along hiking trails, deer trails, and dirt roads. The results of the test show that TREO was completely effective in repelling the Ixodes damini tick.

Because it is known that people wearing a DEET product repellent have been bitten by ticks, a laboratory study with ticks was conducted. The ticks were placed in the centers of filter paper disks either left untreated or ringed by TREO or a DEET product, and the rate at which the ticks crossed the barrier (an untreated or treated ring) monitored. The laboratory test showed TREO to be a more repellent barrier to ticks than the DEET product. Ticks ringed with the DEET products lifted their bodies away from the repellent, but still crossed the treated area relatively quickly, while ticks ringed with TREO all turned back from the barrier at least once and required several attempts before crossing the barrier. Thus, TREO was found to be a true repellent of ticks as not only did TREO discourage initial contact with ticks (the Massachusetts flag test), but also reduced the probability that a tick which might adhere to clothing treated with TREO would climb past a TREO barrier to find an attachment site for blood-feeding. In nature, rather than in the laboratory, it is believed that ticks would simply turn and walk off or drop off a person rather than repeatedly attempting to cross the barrier.

As aforementioned, the preferred embodiment of TREO contains four actives in amounts between .05 and .08 weight percent of a lotion. Testing has shown that the actives alone in concentrations of up to one percent are not as effective as the combined actives in the smaller percentages. Also, subcombinations of the actives in the small amounts proved to be as or nearly as effective as individual actives in the larger concentrations. In particular, Table 2 shows relative alighting percentages of mosquitoes in field and laboratory tests where human arms and paper were treated with the single active, and combinations of the actives in different percentages. The alighting percent should be interpreted as 100% minus the percentage of mosquitoes which turned away from the treated arm or paper.

**Table 2**

| Percent of mosquitoes resting on paper or arms treated single constituent and combination of constituents | | | | |
|---|---|---|---|---|
| Constituent | | | | Alighting % |
| Terpineol | Citronella Oil | Rhodinol | Geraniol | |
| 1% | ---- | ---- | ---- | 12% |
| ---- | 1% | ---- | ---- | 6% |
| ---- | ---- | 1% | ---- | 9% |
| ---- | ---- | ---- | 1% | 12% |
| 1% | 1% | 1% | ---- | 6% |
| 1% | 1% | 1% | 1% | 3% |
| .06% | .05% | .08% | .06% | 3% |
| .06% | .05% | .08% | | 12% |
| .06% | .05% | | .06% | 12% |

From Table 2, it is seen that the four constituent combination is as effective with weight percentages of between .05% and .08% as it is with weight percentages of 1%. Because increased percentages provide little or no advantages, it is preferable to limit each active in TREO to 1% of the lotion. Furthermore, it is believed that the four constituent combination has efficacy as an insect repellent in even lower percentages: each active being at .01% or higher. Also, as shown in Table 2, while not as effective as the four constituent combination, two different three constituent combinations do function as insect repellents. Thus, the combinations of terpineol, citronella oil and geraniol, and terpineol, citronella oil and rhodinol extra are believed to have synergistic efficacy where each active is provided in percentages of at least .01%.

Those skilled in the art will appreciate that the actives in TREO can be carried in different forms. One preferred form is a moisturizer lotion including one or more of glycerin, lanolin alcohol, aloe vera gel, sweet almond oil, propylene glycol, mineral oil, cetyl alcohol, cetyl acetate, and octyl palmitate, to which a fragrance is added. The preferred fragrance is formula #HB-728 of Shaw Mudge & Co., Stamford, Connecticut, which is added to comprise .4% of the moisturizer lotion. The preferred fragrance appears to mask the odor of the actives (which is generally unpleasant to humans) to the human sense of smell, while not adversely affecting the efficacy of the insect repellent to insects.

According to another preferred aspect of the invention, the TREO actives (or preferred subsets) are added to a sun screen lotion of desired SPF level containing actives such as octyl methoxycinnamate, benzophenone-3, or octyl salicylate, to provide a multipurpose lotion; i.e. a sunscreen; a tick/insect repellent; and a moisturising cream.

In accord with another aspect of the invention, the TREO actives (or the preferred subsets) are added in the preferred percentages to a water or alcohol medium to provide an insect repellent spray. The spray is particularly useful for clothing, and either the spray or lotion can be used as an insect repellent for cats, dogs, cattle, sheep, and other animals.

There have been described herein insect repellents which incorporate three or more actives in small percentages into a conveying medium. Thus, while preferred percentages and ranges of actives were described, it will be appreciated that different relative percentages of the actives within those ranges could be utilised, although it is not known whether the resulting combination would be as efficacious as the preferred embodiment. Further, while preferred conveying mediums were described, other mediums including lotions, sprays, and creams could be utilised. In fact, other active ingredients for other purposes, such as suntanning, sunscreening, sunblocking, etc. can be added to the lotions, sprays or creams. Also, while a certain fragrance was described as being used in a particular amount, other fragrances could be utilised, and different amounts of fragrance could be utilised although the other fragrances or different amounts might not mask the odour of the actives as well or as pleasantly as is done in the preferred embodiment.

## Claims

1. An insect repellent formulation suitable for human use, comprising:
(a) a vehicle constituting up to 99.97% of the insect repellent, said vehicle being suitable for application to and use on a human body;
(b) 0.01% to 1% terpineol; and
(c) 0.01% to 1% citronella oil;
together with one or more of rhodinol and geraniol, each of which, when present, is in an amount of 0.01% to 1%;
wherein all listed percentages (%) are by weight.

2. A formulation according to claim 1, which contains 0.06% of said terpineol, 0.05% of said citronella oil and 0.08% of said rhodinol.

3. A formulation according to claim 1, which contains 0.06% of said terpineol, 0.05% of said citronella oil, and 0.06% of said geraniol.

4. A formulation according to any of claims 1 to 3, which contains both said rhodinol and said geraniol.

5. A formulation according to claim 4, wherein said terpincol, said citronella oil, said rhodinol and said geraniol are actives present in said vehicle in relative weights of 6:5:8:6.

6. A formulation according to any of claims 1 to 5, wherein said vehicle comprises any of the following:
a cosmetic moisturiser lotion, a water based vehicle, or an alcohol based vehicle.

7. A formulation according to claim 6, wherein said moisturiser lotion contains a sun screen.

8. A formulation according to any of claims 1 to 7, which further comprises a fragrance.

9. A formulation according to claim 7, which contains 0.4% of said fragrance.

## Patentansprüche

1. Insektenvertreibende Formulierung, die zur Benutzung durch Menschen geeignet ist, umfassend:
(a) eine Trägersubstanz, die bis zu 99,97 % des Insektenvertreibungsmittels ausmacht, wobei die besagte Trägersubstanz zum Aufbringen auf den und zur Benutzung am menschlichen Körper geeignet ist,
(b) 0,01 % bis 1 % Terpineol, und
(c) 0,01 % bis 1 % Citronellöl,
zusammen mit Rhodinol und/oder Geraniol, von denen jedes, wenn enthalten, in einer Menge von 0,01 % bis 1 % enthalten ist; wobei alle aufgeführten Prozentsätze (%) Gewichtsprozente sind.

2. Formulierung nach Anspruch 1, die 0,06 % des besagten Terpineols, 0,05 % des besagten Citronellöls und 0,08 % des besagten Rhodinols enthält.

3. Formulierung nach Anspruch 1, die 0,06 % des besagten Terpineols, 0,05 % des besagten Citronellöls und 0,06 % des besagten Geraniols enthält.

4. Formulierung nach einem der Ansprüche 1 bis 3, die sowohl das besagte Rhodinol als auch das besagte Geraniol enthält.

5. Formulierung nach Anspruch 4, in der das besagte Terpineol, das besagte Citronellöl, das besagte Rhodinol und das besagte Geraniol Wirkstoffe sind, die in der besagten Trägersubstanz mit relativen Gewichten von 6:5:8:6 enthalten sind.

6. Formulierung nach einem der Ansprüche 1 bis 5, in der die besagte Trägersubstanz einen oder mehrere der folgenden Bestandteile umfaßt: eine kosmetische Feuchtigkeitslotion, eine Trägersubstanz auf Wasserbasis oder eine Trägersubstanz auf Alkoholbasis.

7. Formulierung nach Anspruch 6, in der die besagte Feuchtigkeitslotion ein Sonnenschutzmittel enthält.

8. Formulierung nach einem der Ansprüche 1 bis 7, die zusätzlich einen Duftstoff enthält.

9. Formulierung nach Anspruch 7, die 0,4 % des besagten Duftstoffes enhält.

## Revendications

1. Composition anti-insecte pouvant être utilisée par un être humain, comprenant :
(a) un véhicule représentant plus de 99,97 % de la composition anti-insecte, ledit véhicule étant adapté pour être appliqué sur un corps humain;
(b) de 0,01% à 1% de terpinéol ; et
(c) de 0,01% à 1% d'huile de citronnelle;
mélangé avec du rhodinol et/ou du géraniol, chacun étant présent, le cas échéant, à raison de 0,01% à 1%;
dans laquelle tous les pourcentages (%) s'entendent en poids.

2. Composition selon la revendication 1, contenant de 0,06% dudit telpinéol, 0,05 % de ladite huile de citronnelle, et 0,08% dudit rhodinol.

3. Composition selon la revendication 1, contenant de 0,06% dudit terpinéol, 0,05% de ladite huile de citronnelle, et 0,06% dudit géraniol.

4. Composition selon l'une quelconque des revendications 1 à 3, ladite composition contenant à la fois dudit rhodinol et dudit géraniol.

5. Composition selon la revendication 4, dans laquelle ledit terpinéol, ladite huile de citronnelle, ledit rhodinol et ledit géraniol sont des principes actifs présents dans ledit véhicule selon un rapport pondéral relatif de 6:5:8:6.

6. Composition selon l'une quelconque des revendications 1 à 5, dans laquelle ledit véhicule comprend l'un des éléments suivants : une lotion cosmétique hydratante, un véhicule à base d'eau, ou un véhicule à base d'alcool.

7. Composition selon la revendication 6, dans laquelle ladite lotion hydratante contient un écran solaire.

8. Composition selon l'une quelconque des revendications 1 à 7, comprenant de plus du parfum.

9. Composition selon la revendication 7, contenant 0,4% dudit parfum.
